# EUROPEAN PATENT SPECIFICATION

(11) **EP 4 387 695 B1**
(45) Date of publication and mention of the grant of the patent: **07.10.2026**
(21) Application number: 22859221.8
(22) Date of filing: 19.08.2022
(51) Int. Cl.: A61M 16/04, A61B 5/1459, A61B 5/01, A61B 5/1455, A61B 5/00

(54) **ENDOTRACHEAL TUBE**
ENDOTRACHEALTUBUS
TUBE ENDOTRACHÉAL

(30) Priority: 20.08.2021 US 202163235256 P
(43) Date of publication of application: 26.06.2024
(73) Proprietor: Hackensack Meridian Health, Inc., Edison, NJ 08837 (US)
(72) Inventor: SARNOWSKI, Nicholas, Toms River, NJ 08753 (US)
(74) Representative: Schlich
(86) International application number: PCT/US2022/040894
(87) International publication number: WO 2023/023329

(56) References cited:
- WO-A2-2011/115838
- DE-A1- 102009 053 067
- US-A- 5 005 573
- US-A1- 2005 284 483
- US-A1- 2010 288 289
- US-A1- 2012 215 074
- US-A1- 2017 340 216

## Description

### CROSS-REFERENCE TO RELATED APPLICATIONS

This application claims the benefit of U.S. Provisional Patent Application No. 63/235,256, which was filed on August 20, 2021.

### FIELD OF THE INVENTION

The present disclosure relates to endotracheal tubes for use in medical settings and, more particularly, endotracheal tubes including one or more sensors for accurate measurement of patient vital signs and/or physiological parameters while avoiding foreign body complications..

### BACKGROUND OF THE INVENTION

An endotracheal tube can be used in a variety of medical situations during treatment of a patient. For example, endotracheal tubes may be used during general anesthesia, intensive care, and/or emergency medicine treatment for airway management and mechanical ventilation. Traditional endotracheal tubes include a tube and an inflatable cuff coupled at or near the distal end of the tube. The distal end of the tube is configured and dimensioned for insertion into a patient's trachea in order to ensure that the airway is not closed off and that air is able to reach the lungs. The internal diameter for the tube of endotracheal tubes can range in size from about 2 mm to about 10.5 mm, with the size selected based on the patient's body size. For example, smaller sized tubes may be used for pediatric and neonatal patients, while larger sized tubes may be used for adults.

The cuff coupled or secured at or near the distal end of the endotracheal tube is initially maintained in a deflated configuration to allow for insertion of the distal end of the tube into the trachea of the patient. After insertion and positioning of the endotracheal tube at the desired location of the trachea of the patient, the cuff can be inflated to form a seal between the outer surface of the endotracheal tube (i.e., the cuff of the endotracheal tube) and the trachea. The flexibility of the cuff allows for the cuff to conform to and abut the inner wall of the trachea as the cuff is inflated. This seal between the cuff and the inner wall of the trachea helps to create a closed system to allow for the typical increase driving pressures used in mechanical ventilation. The seal between the cuff and trachea also assists in anchoring the endotracheal tube relative to the trachea, thereby reducing or preventing movement of the endotracheal tube and maintaining it in the desired position, e.g.., in a highly vascularized area. In general, when properly positioned, the distal end of an endotracheal tube may be located in the trachea in proximity to the ascending aorta. A ventilator or similar machine can be connected to the endotracheal tube and allows for mechanical ventilation of the patient through the endotracheal tube.

Generally, healthcare professionals may need to monitor one or more vital signs of patients undergoing medical treatment. Such monitoring may be needed to ensure vital signs are at the appropriate levels, and/or may be needed to monitor how patients react to certain medications and/or therapies used for treatment. A variety of sensors may be used to monitor these vital signs. In some instances, one of the vital signs monitored may be the oxygen level of the patient. Pulse oximeters are generally used for monitoring the oxygen level of the patient, and are commonly disposed on a finger, toe, ear, nose and/or forehead of the patient. However, obtaining accurate and consistent oxygen level data with traditional positioning of pulse oximeters may be difficult due to various factors, such as patient movement, patient temperature, or the like. For example, for patients undergoing dialysis, the body temperature can drop down significantly, resulting in difficulty in obtaining oxygen readings.

The following documents are known in the art, namely DE 10 2009 053067 A1, WO 2011/115838 A2 and US 2005/284483 A1.

### SUMMARY OF THE INVENTION

The invention is defined in the appended claims. An exemplary endotracheal tube is provided that includes an elongated tube and an inflatable cuff disposed at or near a distal end of the elongated tube. The inflatable cuff includes one or more sensors disposed on an outer surface. In some embodiments, the one or more sensors can include a pulse oximeter for measurement of oxygen levels of the patient. The endotracheal tube includes a secondary cuff or cover that is positioned over the one or more sensors. In some embodiments, the secondary cuff can cover the entire outer surface of the inflatable cuff. In some embodiments, the secondary cuff only partially covers the outer surface of the inflatable cuff, particularly the one or more sensors and the outer surface of the inflatable cuff adjacent to the one or more sensors. After positioning of the distal end of the endotracheal tube into the trachea of the patient, the cuff can be inflated to create a seal with the inner wall(s) of the trachea.

As the cuff is inflated, the one or more sensors are positioned against the inner wall(s) of the trachea. The secondary cuff or cover protects the one or more sensors from damage during insertion into the trachea and/or during inflation of the cuff, while ensuring that the one or more sensors are appropriately positioned against the inner wall(s) of the trachea. Such positioning allows for accurate oxygenation measurement due to the highly vascularized area of the trachea wall(s). In addition, due to the inflated cuff and the maintained position of the cuff (and sensor(s)) relative to the trachea wall, movement of the patient does not affect or reposition the sensor, ensuring that consistent and accurate measurements of blood oxygen levels are detected as long as the cuff is maintained in the inflated configuration.

In accordance with embodiments of the present disclosure, an exemplary endotracheal tube is provided that includes an elongate tubular member having a proximal end and a distal end, a first cuff positioned about an outer surface of the tubular member, at least one sensor disposed on an outer surface of the first cuff, and a second cuff positioned over the at least one sensor so that the first cuff and the second cuff sandwich the at least one sensor.

In accordance with embodiments of the present disclosure, an exemplary endotracheal tube is provided. The endotracheal tube can include a tubular member extending from a proximal end to a distal end, and defining an outer surface and an inner surface of the tubular member. The endotracheal tube can include a first cuff positioned about a portion of the outer surface of the tubular member, the first cuff defining an inner surface and an outer surface of the first cuff. The endotracheal tube can include a sensor disposed on or adjacent to the outer surface of the first cuff. The endotracheal tube can include a second cuff positioned over the sensor and at least partially over the first cuff, wherein the sensor is disposed between the first and second cuffs.

The inner surface of the first cuff faces towards the outer surface of the tubular member, and the outer surface of the first cuff faces away from the tubular member. In some embodiments, the first cuff can be coupled to the outer surface of the tubular member at or near the distal end of the tubular member. In some embodiments, the first cuff can be inflatable. In some embodiments, the endotracheal tube can include a first inflation tube extending through the tubular member and in communication with an interior of the first cuff for selectively inflating and deflating the first cuff.

In some embodiments, the second cuff can be non-inflatable. In some embodiments, the second cuff can be inflatable, and the first inflation tube can be in communication with an interior of the second cuff for selectively inflating and deflating the second cuff. In some embodiments, the second cuff can be inflatable, and the endotracheal tube can include a second inflation tube extending through the tubular member and in communication with an interior of the second cuff for selectively inflating and deflating the second cuff independently from the first cuff.

The second cuff defines an inner surface and an outer surface of the second cuff, and the sensor is disposed between the first and second cuffs such that the inner surface of the second cuff is positioned immediately adjacent to the sensor. In some embodiments, the sensor can be physically attached to the outer surface of the first cuff without physical attachment to the second cuff. In some embodiments, the sensor can be physically attached to the inner surface of the second cuff without physical attachment to the first cuff. In some embodiments, the sensor can be physically attached to the outer surface of the first cuff and the inner surface of the second cuff.

In some embodiments, the second cuff can be disposed over an entire surface area of the outer surface of the first cuff. In some embodiments, the second cuff can disposed over only a portion of a surface area of the outer surface of the first cuff, and the portion of the surface area is less than 50%. In some embodiments, the portion of the surface area can be less than 25%.

In some embodiments, the sensor can include a single pulse oximeter. In some embodiments, the sensor can include multiple different types of sensors. In some embodiments, the sensor can be configured to move radially outward away from the tubular member during inflation of the first cuff. The distal end of the tubular member can be configured to be positioned within a trachea of a patient, and inflation of the first cuff moves the sensor radially outward away from the tubular member and against a wall of the trachea.

In some embodiments, the first cuff and the second cuff can be fabricated from a same material. In some embodiments, the first cuff and the second cuff can be fabricated from a biocompatible material. In some embodiments, the endotracheal tube can include an electrical conductor coupled to the sensor and extending through a portion of the tubular member.

In accordance with embodiments of the present disclosure, an exemplary method of measuring one or more vital signs or physiological parameters of a patient is provided. The method includes inserting a distal end of an endotracheal tube into a trachea of a patient. The endotracheal tube includes a tubular member extending from a proximal end to a distal end, and defining an outer surface and an inner surface of the tubular member, a first cuff positioned about a portion of the outer surface of the tubular member, the first cuff defining an inner surface and an outer surface of the first cuff, a sensor disposed on or adjacent to the outer surface of the first cuff, and a second cuff positioned over the sensor and at least partially over the first cuff. The sensor is disposed between the first and second cuffs. The method includes expanding the first cuff of the endotracheal tube to move the sensor radially outward away from the tubular member and against a wall of the trachea. The method includes measuring one or more vital signs or physiological parameters of the patient with the sensor.

In some embodiments, the method can include inflating the first cuff with a fluid to move the sensor radially outward away from the tubular member and against the wall of the trachea, the first cuff forming a seal with the wall of the trachea. In some embodiments, the method can include inflating the second cuff with a fluid to move the sensor radially outward away from the tubular member and against the wall of the trachea. In some embodiments, the second cuff can define an inner surface and an outer surface of the second cuff, and the sensor can be disposed between the first cuff and the second cuff such that the inner surface of the second cuff is positioned immediately adjacent to the sensor. In some embodiments, measuring the one or more vital signs or physiological parameters of the patient with the sensor can include measuring an oxygen saturation of a patient when the first cuff is inflated and the sensor is positioned against the wall of the trachea.

In accordance with embodiments of the present disclosure, an exemplary tracheostomy tube is provided. The tracheostomy tube can include a tubular member extending from a proximal end to a distal end, and defining an outer surface and an inner surface of the tubular member. The tracheostomy tube can include a first cuff positioned about a portion of the outer surface of the tubular member, the first cuff defining an inner surface and an outer surface of the first cuff. The tracheostomy tube can include a sensor disposed on or adjacent to the outer surface of the first cuff. The tracheostomy tube can include a second cuff positioned over the sensor and at least partially over the first cuff. The sensor is disposed between the first and second cuffs.

In some embodiments, the tracheostomy tube can include a tracheal collar coupled to the proximal end of the tubular member.

Objects and features will become apparent from the following detailed description considered in conjunction with the accompanying drawings. It is to be understood, however, that the drawings are designed as an illustration only and not as a definition of the limits of the present disclosure.

### BRIEF DESCRIPTION OF THE DRAWINGS

To assist those of skill in the art in making and using the disclosed endotracheal tube, reference is made to the accompanying figures, wherein:
**FIG. 1** is a diagrammatic, perspective view of an exemplary endotracheal tube in accordance with the present disclosure, including a cuff in a deflated/collapsed configuration and a sensor incorporated onto the cuff;
**FIG. 2** is a diagrammatic, perspective view of an exemplary endotracheal tube of FIG. 1, including a cuff in an inflated/expanded configuration;
**FIG. 3** is a diagrammatic, perspective view of an exemplary endotracheal tube in accordance with the present disclosure, including a cuff in an inflated/expanded configuration and a sensor incorporated onto the cuff;
**FIG. 4** is a perspective view of an exemplary tracheostomy tube in accordance with the present disclosure, including a cuff in a deflated/collapsed configuration and a sensor incorporated onto the cuff;
**FIG. 5** is a perspective view of an exemplary tracheostomy tube in accordance with the present disclosure, including a cuff in an inflated/expanded configuration and a sensor incorporated onto the cuff; and
**FIG. 6** is a detailed, perspective view of an exemplary tracheostomy tube in accordance with the present disclosure, including a cuff in an inflated/expanded configuration and a sensor incorporated onto the cuff.

### DETAILED DESCRIPTION OF THE INVENTION

As used herein, the term "proximal," when used in connection with a component of an endotracheal tube, refers to the end of the component closest to the physician when the endotracheal tube is inserted in a patient. The term "proximal" can also refer to the portion of the endotracheal tube that lies outside of the patient after installation.

As used herein, the term "distal," when used in connection with a component of an endotracheal tube, refers to the end of the component furthest from the physician when the endotracheal tube is inserted in a patient. The term "distal" can also refer to the portion of the endotracheal tube that lies inside of the trachea of the patient after installation.

As used herein, the terms "trailing" and "leading" are to be taken as relative to the operator (e.g., physician) of the endotracheal tube. "Trailing" is to be understood as relatively close to the operator, and "leading" is to be understood as relatively further away from the operator.

Endotracheal tubes (ETTs) are used as a means to provide ventilator support in a variety of medical situations, such as during surgery or COVID-19 treatment.

The exemplary endotracheal tubes discussed herein can be equipped with a variety of one or more sensors. Although a pulse oximeter is discussed as the sensor incorporated onto the endotracheal tube and used to measure oxygen saturation, it should be understood that other suitable sensors may be used in combination, or instead of, the pulse oximeter. The one or more sensors capable of incorporated into the endotracheal tube assembly can include, e.g., ultrasound transducers, temperature sensors, blood gas sensors (e.g., CO₂, and infrared sensors to measure tissue oximetry), air flow sensors, capnography sensors (to measure CO₂ gas), sensors for an esophageal electrocardiogram, one or more sensors for sampling of exhaled gases (for example, nitrous or nitric oxide), sensors for measuring humidity, combinations thereof, or the like. However, the list of sensor types provided herein is nonlimiting, and any sensor known to those of ordinary skill in the art that provides information of interest to an attending physician and/or healthcare professional (such as information regarding patient vital signs and/or physiological parameters) can be incorporated into the endotracheal tube. In some embodiments, information from the one or more sensors can be used to measure metabolic rates, e.g., the percentage consumption of oxygen and percentage production of CO₂ provide a measure of metabolism which can be used to monitor patient status in critical care situations.

The endotracheal tube discussed herein can be used in any medical situation in which it is desirable to ensure that the patient airway is maintained and where measuring a vital sign and/or physiological parameter of the patient (such as, but not limited to, oxygen saturation) would be beneficial. In some embodiments, the endotracheal tube can be used in the practice of surgery where a patient is to be ventilated and/or treated with anesthesia. In some embodiments, the endotracheal tube can be used in conjunction with a cryosurgical procedure. In some embodiments, the endotracheal tube can be used for monitoring of patients in critical care situations.

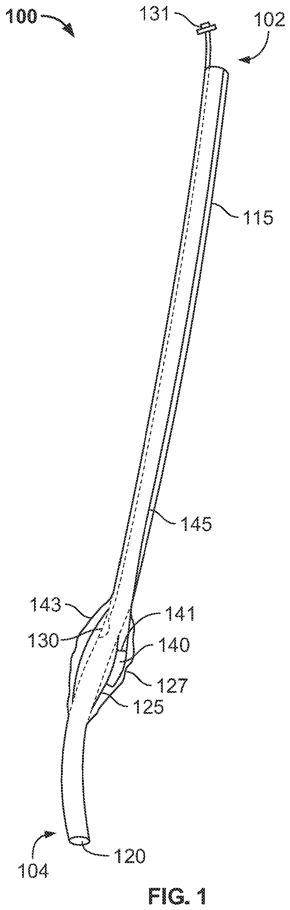

**FIG. 1** is a diagrammatic, perspective view of an exemplary endotracheal tube 100 in accordance with the present disclosure. The endotracheal tube 100 includes an elongated tube or tubular member 115 that generally extends between a proximal end 102 and a distal end 104. In some embodiments, the tubular member 115 can be fabricated from, e.g., a medical grade plastic material, a medical grade polyvinyl chloride (PVC), or the like. The tubular member 115 defines an outer surface and an inner channel or passage 120 that extends along the entire length of the tubular member 115 from the proximal end 102 to the distal end 104.

The endotracheal tube's size refers to its internal diameter in millimeters (mm). The ETT will typically list both the inner diameter and outer diameter on the tube (for example, a 6.0 endotracheal tube will list both the internal diameter, ID 6.0, and outer diameter, OD 8.8). The narrower the tube, the greater resistance to gas flow. The largest tube that is appropriate for the patient is generally selected; this is critically important to the spontaneously breathing patient who will have to work harder to overcome the increased resistance of a smaller ETT (a size 4 ETT has 16 more times resistance to gas flow than a size 8 ETT).

The passage 120 defines a uniform (or substantially uniform) diameter that can range from about, e.g., 2-10.5 mm inclusive, 2-10 mm inclusive, 2-9 mm inclusive, 2-8 mm inclusive, 2-7 mm inclusive, 2-6 mm inclusive, 2-5 mm inclusive, 2-4 mm inclusive, 2-3 mm inclusive, 3-10.5 mm inclusive, 4-10.5 mm inclusive, 5-10.5 mm inclusive, 6-10.5 mm inclusive, 7-10.5 mm inclusive, 8-10.5 mm inclusive, 9-10.5 mm inclusive, 10-10.5 mm inclusive, 2 mm, 3 mm, 4 mm, 5 mm, 6 mm, 7 mm, 8 mm, 9 mm, 10 mm, 10.5 mm, or the like, depending on the patient's body size. The passage 120 allows fluid communication between the ambient atmosphere and the patient's lungs, as well as a ventilator for assistance with mechanical ventilation. The tubular member 115 can generally define a flexible and/or curved shape such that the distal end 104 of the endotracheal tube 100 can be inserted into the trachea via the mouth. In some embodiments, other shapes of the tubular member 114 can be used for insertion through the nose and/or a tracheotomy.

The elongate tubular member 115 can be in the form of a flexible, curved, hollow tube. However, the endotracheal tube 100 and/or the tubular member 115 can take any shape and/or size known to those of ordinary skill in the art. The tubular member 115 can be flexible or rigid. The tubular member 115 (and/or any other component associated with the endotracheal tube 100) can be constructed of any suitable material used for endotracheal tubes known in the industry, e.g., any biocompatible material. In some embodiments, the materials that can be used to fabricate the tubular member 115 can include, but are not limited to, polyvinylchloride, latex, silicone, rubber, or other materials readily apparent to those of ordinary in the art. In some embodiments, the endotracheal tube 100 can be fabricated from a clear biocompatible material. In some embodiments, the other components of the endotracheal tube 100 (e.g., the cuffs 125, 127) can be fabricated from the same material as the tubular member 115.

The typical depth of the endotracheal tube is 23 cm for men and 21 cm for women, measured at the central incisors. The average size of the tube for an adult male is 8.0, and for an adult female is 7.0; this is somewhat an institution dependent practice. Pediatric tubes are sized using the equation: size = ((age/4) +4) for uncuffed ETTs, with cuffed tubes being one-half size smaller.[Aker, J. AANA J. (2008) 76 (4): 293-300] Typically a pediatric ETT is taped at a depth of 3x the tube size in a child (i.e., a 4.0 ETT commonly gets taped at around 12 cm depth). [Ahmed, RA and Boyer, TJ. Endotreacheeal Tube. StatPearls Publishing 2022, Jan. PMID: 30969569].

The endotracheal tube 100 includes an inflatable, flexible cuff 125 disposed at or near the distal end 104 of the tubular member 115. A cuff is an inflatable balloon at the distal end of the ETT. A defective balloon will result in a loss of ability to protect the airway from aspirate and may make mechanical ventilation difficult.

As illustrated in **FIG. 1****,** the cuff 125 can be disposed spaced from the distal end 104 (e.g., between a central point of the tubular member 115 and the distal end 104). In some embodiments, the cuff 125 can be integrally formed with the tubular member 115. In some embodiments, the cuff 125 can be formed separately from the tubular member 115 and adhered to the outer surface of the tubular member 115 in a sealed manner such that that cuff 125 can be selectively inflated and deflated by a user. The cuff 125 generally extends circumferentially around the tubular member 115 such that, in an inflated configuration, the cuff 125 creates a circumferential seal with the inner walls of the trachea of the patient. In some embodiments, the cuff 125 can be fabricated from a flexible, medical grade material, such as plastic or PVC, to allow for inflation of the cuff 125.

In particular, the cuff 125 can be inflated to form a seal between the outer surface of the endotracheal tube 100 and/or the outer surface of the cuff 125 and the inner wall of the trachea. The endotracheal tube 100 includes an inflation tube 130 in fluid communication with the interior of the cuff 125 through which material (e.g., a fluid, air, or the like) may be selectively introduced into and removed from the interior of the cuff 125. In some embodiments, the inflation tube 130 can be built into the wall of the tubular member 115 such that the tube 130 fluidly connects with the interior of the cuff 125 for inflation and deflation of the cuff 125. In some embodiments, the inflation tube 130 can extend along an inner wall of the passage 120 of the tubular member 115 and fluidly connects with the interior of the cuff 125. The material passed through the inflation tube 130 can be acoustically transmissive. The proximal end of tube 130 can extend from the proximal end 102 of the tubular member 115, and can be equipped with a valve 131 which can be used to control the flow of material into and out of the cuff 125.

To facilitate placement through the vocal cords and to provide improved visualization ahead of the tip, the ETT has an angle or slant known as a bevel. As the endotracheal tube approaches the cords, the left-facing bevel provides an optimal view.

Murphy's eye is another opening in the tube positioned in the distal lateral wall. If the distal end of the ETT should become obstructed by the wall of the trachea or by touching the carina of the trachea (meaning a ridge at the base of the trachea that separates the openings of the right and left main bronchi (meaning the large air passages that lead from the trachea to the lungs), gas flow can still occur via Murphy's eye. This prevents complete obstruction of the tube. The carina is the most sensitive area of the trachea and larynx for triggering a cough reflex.

ETT connectors attach the ETT to the mechanical ventilator tubing or a bag valve mask (BVM), sometimes referred to as an Ambu bag, which is a handheld tool that is used to deliver positive pressure ventilation to any subject with insufficient or ineffective breaths. It consists of a self-inflating bag, one-way valve, mask, and an oxygen reservoir.

The endotracheal tube 100 of the present disclosure includes one or more sensors 140 positioned on the exterior or outer surface of the cuff 125 (e.g., outside of the inner volume defined by the cuff 125). The one or more sensors 140 can be used to detect and transmit one or more vital signs and/or physiological parameters associated with a patient. In some embodiments, one sensor 140 can be used to detect and transmit more than one type of vital sign and/or physiological parameter associated with the patient. In some embodiments, the one or more sensors 140 can be connected to a receiving station, computing device, or the like, via wires (e.g., electric conductor(s) 145) passing through the passage 120 and/or a wall of the tubular member 115. In some embodiments, the one or more sensors 140 can wirelessly transmit the measured or detected data to the external receiving station and/or computing device. For example, the sensor 140 can be equipped with wireless transmission capability, and may be used to send/receive signals from the sensor 140 to a suitable external device. In some embodiments, the one or more sensors 140 can be compatible with other medical devices, such as X-ray and/or MRI scanners. For example, the sensors 140 can include an outer coating and/or shielding material 141 that prevents interference with external equipment.

In one embodiment, the sensor 140 can be a pulse oximeter for detection of the oxygen level of the patient. In some embodiments, the pulse oximeter sensor can rely on, e.g., red and infrared light emitting diodes to measure deoxygenated and oxygenated hemoglobin to determine the percentage of oxygen in red blood cells. The sensor 140 can be positioned on the cuff 125 at a central or substantially central position of the cuff 125. For example, the sensor 140 can be positioned at a midpoint between the top and bottom areas of the cuff 125 (e.g., the areas connecting the cuff 125 to the tubular member 120). Such positioning of the sensor 140 relative to the cuff 125 can ensure that when the cuff 125 is inflated, the sensor 140 (or a majority of the surface area of the sensor 140) is positioned against the inner wall of the trachea.

In some embodiments, the sensor 140 can be directly coupled to the outer surface of cuff 125 by gluing, welding, or any other suitable techniques. The coupling of the sensor 140 to the cuff 125 can be performed in a flexible manner to allow for at least partial movement of the sensor 140 as the cuff 125 expands during inflation and contracts during deflation. For example, flexible adhesive can be used to adhere the sensor 140 to the cuff 125, with the adhesive allowing for at least partial movement of the sensor 140 as the cuff 125 inflates and deflates, while maintaining the desired position of the sensor 140 relative to the cuff 125. In some embodiments, the sensor 140 can be disposed on or immediately adjacent to the exterior surface of the cuff 125 without being directly attached to the cuff 125. For example, the sensor 140 can be coupled to the second cuff 127 (discussed below) without being coupled to the cuff 125. In some embodiments, the sensor 140 can be attached to the endotracheal tube 100 by any method or material known to those of ordinary skill in the art. For example, in some embodiments, the sensor 140 can be permanently affixed to the outer surface of first cuff 125 by heat sealing and/or adhesive. In some embodiments, the sensor 140 can be permanently affixed to the inner surface of second cuff 127 by heat sealing and/or adhesive, or can be free-floating between the two cuffs 125, 127 (e.g., not directly coupled to either cuff).

As illustrated in **FIG. 1****,** the endotracheal tube 100 can include a protective layer (e.g., a second cuff 127) disposed over the sensor 140. The second cuff 127 is dimensioned greater in diameter than the first cuff 125 based on its positioning over the first cuff 125. The second cuff 127 can be secured to the tubular member 115 to create a seal therebetween, and to create a seal in the space between the first and second cuffs 125, 127. In some embodiments, the second cuff 127 can be fabricated from the same material as the first cuff 125. For example, the cuffs 125, 127 can be fabricated from a flexible, biocompatible material, e.g., a biocompatible plastic, such as polyvinyl chloride (PVC), or the like. Any suitable material can be used to inflate the cuff 125 (and/or the cuff 127 in embodiments in which the cuff 127 is also inflated). In some embodiments, the fluid(s) used to inflate the cuff 125 and/or cuff 127 can include, e.g., water, saline solution, buffer solutions, acoustical gels, or the like. When inflated, the cuff 125 and/or cuff 127 forms a seal against the inner wall of the trachea. Although **FIG. 1** illustrates the cuff 125 as symmetrically arranged around tubular member 115, it should be understood that in some embodiments, the cuff can be asymmetrically arranged around the tubular member 115. The cuff 125 is attached to the tubular member 115 such that the cuff 125 retains the material used to inflate it. The endotracheal tube 100 therefore includes an inner cuff (i.e., first cuff 125) and an outer cuff (e.g., second cuff 127) disposed relative to the sensor 140. The second cuff 127 can be disposed over the sensor 140 and extends over the entire surface area of the first cuff 125. The sensor 140 is therefore sandwiched between the first and second cuffs 125, 127.

In some embodiments, the second cuff 127 can extend over the entire surface area of the first cuff 125 (e.g., 100% of the first cuff 125). In some embodiments, the second cuff 127 can extend over a majority of the exterior surface area of first cuff (e.g., over more than about 50%, over more than about 60%, over more than about 70%, over more than about 80%, over more than about 90%, over more than about 95%, or the like). In some embodiments, the outward facing surface of the sensor 140 can be coupled to the inner surface of the second cuff 127 via, e.g., adhesive, welding, or the like. In some embodiments, the inward facing surface of the sensor 140 can be coupled to the outer surface of the first cuff 125 and the outward facing surface of the sensor 140 can be coupled to the inner surface of the second cuff 127 to prevent or reduce air between the sensor 140 and the second cuff 127. In some embodiments, the inward facing surface of the sensor 140 can be coupled to the outer surface of the first cuff 125 without being coupled to the second cuff 127, and the second cuff 127 can stretch over the sensor 140 during inflation of the first cuff 125 to prevent or minimize a layer of air between the sensor 140 and the second cuff 127. Although one sensor 140 is illustrated, if multiple sensors 140 are used, the sensors 140 can be disposed in a spaced manner relative to each other between the cuffs 125, 127.

In some embodiments, the second cuff 127 can be non-inflatable and is formed from a flexible material that allows for the second cuff 127 to stretch and remain positioned over the sensor 140 as the first cuff 125 is inflated. In some embodiments, the second cuff 127 can be inflatable via the same or similar means (or via a separate mechanism) as the first cuff 125. In such embodiments, the second cuff 127 can simultaneously or substantially simultaneously inflate as the first cuff 125 is inflated. For example, as illustrated in FIG. 1, in the collapsed/deflated condition or configuration, both the first and second cuffs 125, 127 remain relatively close to the outer surface of the tubular member 115, and the sensor 140 remains between the cuffs 125, 127 in a compact manner. The deflated cuffs 125, 127 allow for insertion of the distal end 104 of the endotracheal tube 100 into the trachea of the patient in the desired orientation to allow for ventilation of the patient, and measurement of one or more vital signs and/or physiological parameters of the patient. For example, a GLIDESCOPE^{™} (available from Verathon Inc.) can be used to assist in orienting and positioning the sensor 140 on the left side of the trachea of the patient to ensure that the sensor 140 is positioned adjacent to a highly vascularized area of the trachea after inflation of the cuff 125 (and/or cuff 127).

The endotracheal tube 100 can include one or more inflation tubes 130. In some embodiments, a single inflation tube 130 can be used to inflate the cuffs 125, 127. In some embodiments, separate inflation tubes 130 can be used to inflate each of the respective cuffs 125, 127 to allow for independent inflation and deflation control. In some embodiments, the inflation tube 130 can be formed as an integral part of the tubular member 115. In some embodiments, the inflation tube 130 can be formed as a separate tube that runs inside of the passage 120. The proximal end of the inflation tube 130 can remain outside of the patient after installation of the endotracheal tube 100, while the distal end of the inflation tube 130 remains inside of the cuff 125. Thus, the inflation tube 130 provides fluid communication from the outside of the patient to the interior of the cuff 125. The proximal end of the inflation tube 130 can be equipped with a valve 131 to selectively control/regulate the flow of material into and out of the cuff 125 (and/or the cuff 127).

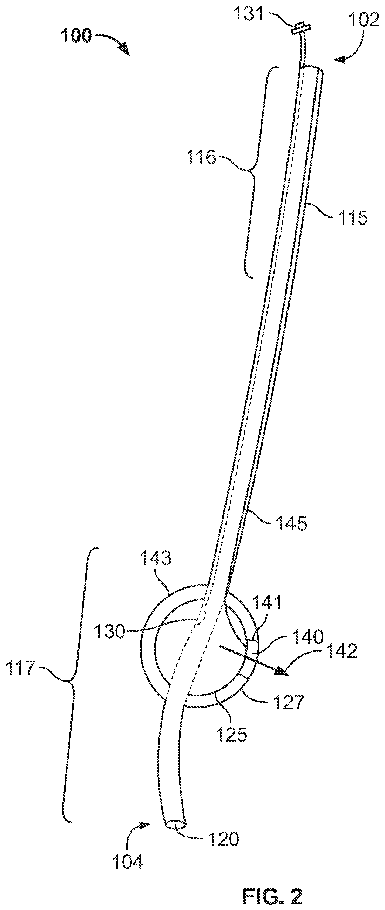

**FIG. 2** illustrates the endotracheal tube 100 with the cuff 125 (and/or the cuff 127) in an expanded/inflated configuration. In particular, either the cuff 125, or both the cuffs 125, 127, can be expanded/inflated as illustrated in **FIG. 2****.** Inflating the inner or first cuff 125 causes the sensor 140 to move radially outward in the direction of arrow 142 toward a tracheal wall such that the sensor 140 is pushed outward and against the tracheal wall. As the first cuff 125 inflates, it expands to move the sensor 140 radially outward toward the tracheal wall. The second cuff 127 simultaneously stretches outward to accommodate the expansion of the first cuff 125. In some embodiments, the second cuff 127 can also be inflated (or at least partially inflated) such that the inner surface of the second cuff 127 is positioned spaced from the outer surface of the first cuff 125 (see, e.g., **FIG. 2**). Such expansion and/or inflation of the second cuff 127 creates a substantially uniform space between the first and second cuffs 125, 127, resulting in a substantially uniform diameter of the second cuff 127. Such uniform diameter can provide for an improved seal with the surrounding tracheal wall.

In some embodiments, the second cuff 127 does not inflate and instead only stretches to accommodate inflation of the first cuff 125. In such embodiments, the inner surface of the second cuff 127 is spaced from the outer surface of the first cuff 125 at the sensor 140, but can be positioned adjacent to each other at one or more areas spaced from the sensor 140. For example, the opposing side 143 of the cuff assembly (i.e., cuffs 125, 127) can include one or more sections of the second cuff 127 positioned adjacent or against the first cuff 125. In some embodiments, the second cuff 127 can be large enough to accommodate the increased volume of first cuff 125 during/after inflation.

In the inflated/expanded configuration, the inner surface of the second cuff 127 remains immediately adjacent to or against the outward facing surface of the sensor 140, such that the sensor 140 and the tracheal wall are positioned immediately adjacent to each other except for the second cuff 127 positioned therebetween. With the sensor 140 being pushed toward and maintained against the tracheal wall, a more consistent and accurate oxygen saturation reading can be obtained. Greater accuracy in the measurement of oxygenation by the endotracheal tube 100 can be provided due to the presence of the large vessels and vasculature near the wall of the trachea. With constant contact with the tracheal mucosa, the pulse oximetry measurement will generally not be lost, unlike using a traditional peripheral pulse oximeter where outside effects may skew the readings (e.g., the use of vasopressors, targeted temperature management protocol, or the like). In addition, even in instances where the body temperature of the patient is low (such as during dialysis), the positioning of the sensor 140 against the tracheal wall can continue to provide accurate and consistent measurements.

The second cuff 127 can provide protection to the pharynx and/or trachea during insertion of the endotracheal tube from the sensor 140. With the sensor 140 sandwiched between the two cuffs 125, 127, the extra protective layer of the second cuff 127 can provide protection from any degradation of the tracheal wall. The sandwiched cuff 125, 127 configuration can also protect the sensor 140 from being dislodged from the tubular member 115 and lost in the pulmonary circuit. For example, if the cuff 125 and/or cuff 127 ruptures, the connection between the sensor 140 and the cuffs 125, 127 can prevent escape of the sensor 140 from the endotracheal tube 100 assembly.

Still with reference to **FIG. 2****,** the endotracheal tube 100 can generally include a proximal portion 116 and a distal portion 117, with the proximal portion 116 remaining outside of the patient after installation and the distal portion 117 being installed within the tracheal of the patient. The proximal portion 116 can exit either the nasal cavity or the mouth of the patient when installed in the trachea of a patient. In some embodiments, the proximal portion 116 can exit through a tracheostomy. When installed, the passage 120 provides fluid communication from the outside of the patient to the lungs. The proximal portion 116 of the endotracheal tube 100 can be attached to any external device typically used to introduce gas into the lungs of the patient, e.g., a bag ventilator, a mechanical ventilator, combinations thereof, or the like.

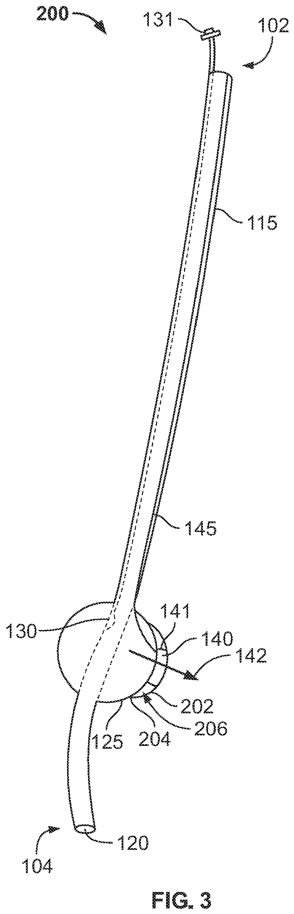

**FIG. 3** is a diagrammatic, perspective view of an exemplary endotracheal tube 200 of the present disclosure. The endotracheal tube 200 can be substantially similar to the endotracheal tube 100, except for the distinctions noted herein. As such, same reference numbers refer to same structures. According to the inventino, rather than including a second cuff 127 that encircles or covers the entire surface area of the first cuff 125, the endotracheal tube 200 includes a second cuff 202 that only covers a portion of the first cuff 125.

The second cuff 202 is dimensioned smaller than the first cuff 125 and, particularly, is dimensioned to cover at least the sensor 140. The second cuff 202 includes a perimeter 204 edge that is attached or coupled to the outer surface of the first cuff 125 to form a pocket 206 between the first and second cuffs 125, 202 encasing the sensor 140. In some embodiments, the perimeter 204 edge can be, e.g., circular, rectangular, square, or the like. The second cuff 202 therefore only covers a small surface area of the first cuff 125 (e.g., less than 50%, less than 40%, less than 30%, less than 25%, or the like).

The inner surface of the second cuff 202 remains substantially adjacent to the outward facing surface of the sensor 140 to ensure proper positioning of the sensor 140 against the tracheal wall after inflation of the first cuff 125. In some embodiments, the second cuff 202 can be non-inflatable, inflatable, or both. The sensor 140 therefore remains sandwiched between the two cuffs 125, 202. In some embodiments, one pocket 206 can be configured and dimensioned to accommodate two or more sensors 140. In some embodiments, separate cuffs 202 can be used to encase separate sensors 140 associated with the endotracheal tube 200.

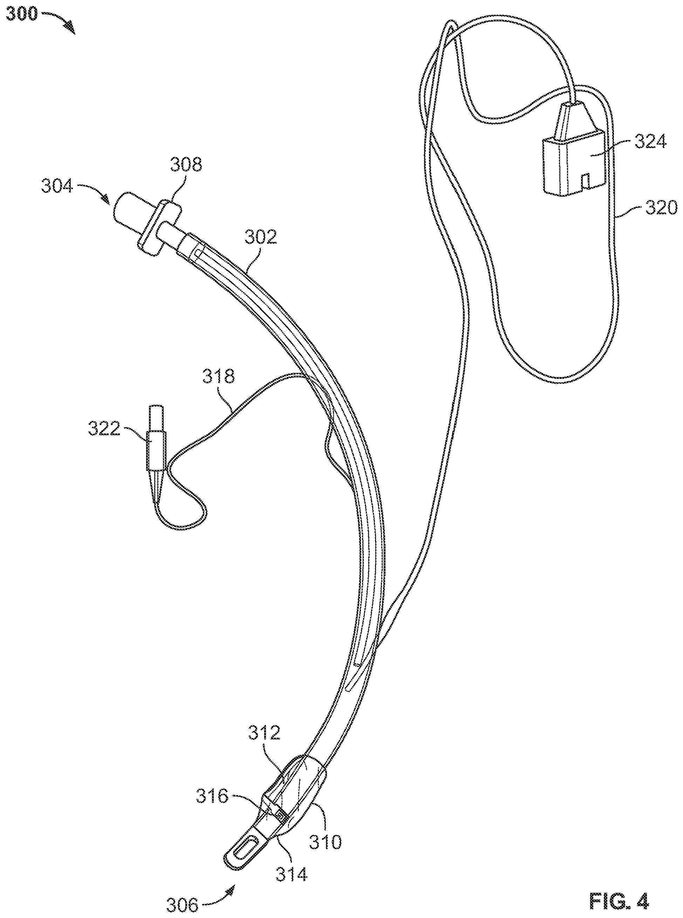

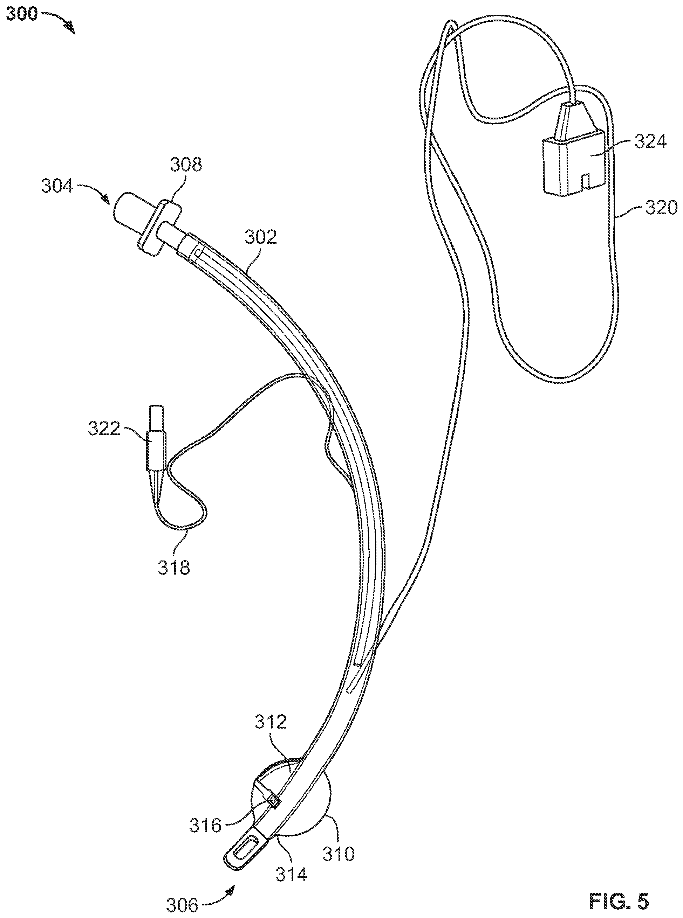

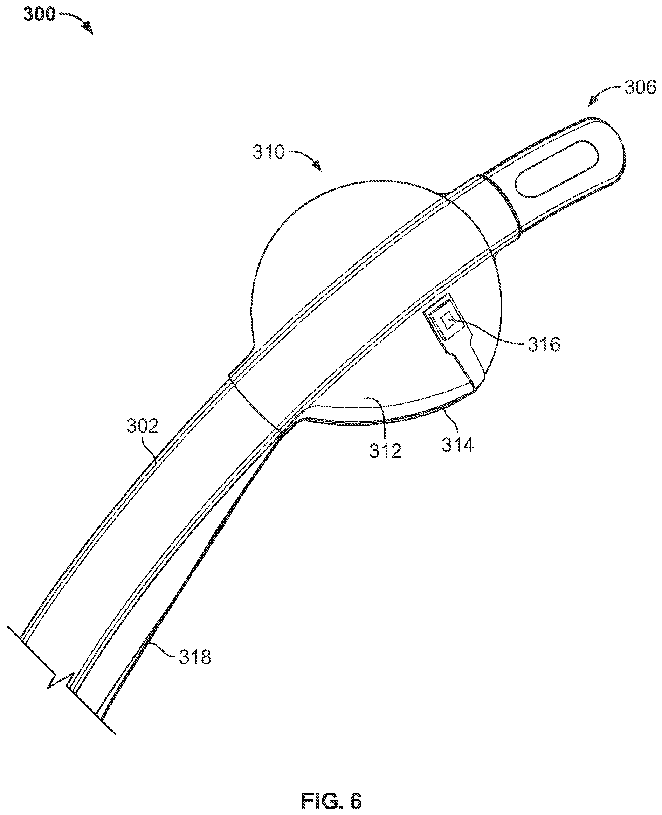

In some embodiments, a similar design as used with the endotracheal tube 100 can be incorporated into other supportive medical equipment. For example, **FIGS. 4-6** illustrate an exemplary tracheostomy tube 300 that includes a dual cuff design with a sensor for monitoring one or more vital signs or physiological parameters of a patient.

The tube 300 generally includes an elongated tubular member 302 that extends between a proximal end 304 and a distal end 306. At or near the proximal end 304, the tube 300 can include a tracheal collar 308 (or an adapter configured to engage with a tracheal collar). At or near the distal end 306, the tube 300 includes a cuff assembly 310 including a first cuff 312 (e.g., an inner cuff) and a second cuff 314 (e.g., an outer cuff). The cuff 312 is positioned around a portion of the outer surface of the tubular member 302. The first cuff 312 can be inflatable such that the cuff 312 can be selectively inflated or deflated for positioning and maintaining the position of the tubular member 302 within the trachea of the patient.

A sensor 316 is positioned on or adjacent to an outer surface of the cuff 312, and the second cuff 314 is positioned over at least the sensor 316 and at least a portion of the first cuff 312. In some embodiments, the second cuff 314 can cover the sensor 316 and the immediately adjacent areas of the first cuff 312 surrounding the sensor 316. In some embodiments, the second cuff 314 can cover the sensor 316 and about 50% of the surface area of the first cuff 312. In some embodiments, the second cuff 314 can cover the sensor 316 and about 75% of the surface area of the first cuff 312. In some embodiments, the second cuff 314 can cover the sensor 316 and about 100% of the surface area of the first cuff 312. The sensor 316 is thereby disposed between the first and second cuffs 312, 314. Although one sensor 316 is illustrated, it should be understood that multiple sensors could be incorporated into the tube 300.

In some embodiments, one or more wires 318, 320 can pass through the interior of the tubular member 302 and/or on the outside of the tubular member 302 for transmission of data/signals to/from the sensor 316 and to/from a computing or processing device (not shown). Each wire 318, 320 can include an electrical connector 322, 324 at a distal end to allow for electrical connection to the computing or processing device. Data can thereby be received from the sensor 316, and the computing or processing device can analyze and display the data to the medical professional.

While exemplary embodiments have been described herein, it is expressly noted that these embodiments should not be construed as limiting, but rather that additions and modifications to what is expressly described herein also are included within the scope of the present disclosure. Moreover, it is to be understood that the features of the various embodiments described herein are not mutually exclusive and can exist in various combinations and permutations, even if such combinations or permutations are not made express herein, without departing from the scope of the present disclosure.

## Claims

1. An endotracheal or tracheostomy tube, comprising:
a tubular member extending from a proximal end to a distal end, and defining an outer surface and an inner surface of the tubular member;
a first cuff positioned about a portion of the outer surface of the tubular member, the first cuff defining an inner surface and an outer surface of the first cuff;
a sensor disposed on or adjacent to the outer surface of the first cuff; and
a second cuff, positioned over the sensor, that only covers a portion and does not encircle or cover the entire surface area of the first cuff and,
wherein the sensor is disposed between the first and second cuffs.

2. The endotracheal tube of claim 1, wherein the inner surface of the first cuff faces towards the outer surface of the tubular member, and the outer surface of the first cuff faces away from the tubular member, and/or the endotracheal tube comprises a first inflation tube extending through the tubular member and in communication with an interior of the first cuff for selectively inflating and deflating the first cuff.

3. The endotracheal tube of claim 1, wherein:
the first cuff is coupled to the outer surface of the tubular member at or near the distal end of the tubular member; or
the first cuff is inflatable; or
the second cuff is non-inflatable.

4. The endotracheal tube of claim 2, wherein:
the second cuff is inflatable, and the first inflation tube is in communication with an interior of the second cuff for selectively inflating and deflating the second cuff; or
the second cuff is inflatable, and the endotracheal tube comprises a second inflation tube extending through the tubular member and in communication with an interior of the second cuff for selectively inflating and deflating the second cuff independently from the first cuff.

5. The endotracheal tube of claim 1, wherein the second cuff defines an inner surface and an outer surface of the second cuff, and wherein the sensor is disposed between the first and second cuffs such that the inner surface of the second cuff is positioned immediately adjacent to the sensor, and wherein the second cuff is configured to stretch over the sensor during inflation of the first cuff, and wherein the second cuff is further configured to prevent or minimize a layer of air between the sensor and the second cuff.

6. The endotracheal tube of claim 1, wherein:
the sensor is physically attached to the outer surface of the first cuff without physical attachment to the second cuff; or
the sensor is physically attached to the inner surface of the second cuff without physical attachment to the first cuff; or
the sensor is physically attached to the outer surface of the first cuff and the inner surface of the second cuff; or
the second cuff is disposed over an entire surface area of the outer surface of the first cuff.

7. The endotracheal tube of claim 1, wherein the second cuff comprises a perimeter edge that is coupled to the outer surface of the first cuff to form a pocket, and wherein the portion of the surface area is less than 50%, and wherein the portion of the surface area is less than 25%.

8. The endotracheal tube of claim 1, wherein:
the sensor comprises a single pulse oximeter; or
the sensor comprises multiple different types of sensors.

9. The endotracheal tube of claim 1, wherein the sensor is configured to move radially outward away from the tubular member during inflation of the first cuff, and optionally wherein the distal end of the tubular member is configured to be positioned within a trachea of a patient, and inflation of the first cuff moves the sensor radially outward away from the tubular member and against a wall of the trachea.

10. The endotracheal tube of claim 1, wherein the first cuff and the second cuff are fabricated from a same material.

11. The endotracheal tube of claim 1, wherein the first cuff and the second cuff are fabricated from a biocompatible material.

12. The endotracheal tube of claim 1, comprising an electrical conductor coupled to the sensor and extending through a portion of the tubular member.

13. An endotracheal tube assembly for measuring one or more vital signs or physiological parameters of a patient, the assembly comprising:
a tubular member extending from a proximal end to a distal end, and defining an outer surface(i) a first cuff positioned about a portion of the outer surface of the tubular member, the first cuff defining an inner surface and an outer surface of the first cuff, (ii) a sensor disposed on or adjacent to the outer surface of the first cuff, and (iii) a second cuff positioned over the sensor wherein the second cuff is disposed over only a portion of a surface area of the outer surface and does not encircle or cover the entire surface area of the first cuff, wherein the sensor is disposed between the first and second cuffs;
wherein the first cuff is configured to expand to move the sensor radially outward away from the tubular member and against a wall of the trachea when the endotracheal tube is inserted into the trachea; and
wherein the sensor is configured to measure one or more vital signs or physiological parameters of the patient.

14. The endotracheal tube assembly of claim 13, wherein:
the first cuff is configured to inflate with a fluid to move the sensor radially outward away from the tubular member and against the wall of the trachea, the first cuff forming a seal with the wall of the trachea; or
the second cuff is configured to inflate with a fluid to move the sensor radially outward away from the tubular member and against the wall of the trachea.

15. The endotracheal tube assembly of claim 13, wherein:
the second cuff defines an inner surface and an outer surface of the second cuff, and wherein the sensor is disposed between the first cuff and the second cuff such that the inner surface of the second cuff is positioned immediately adjacent to the sensor; or
the one or more vital signs or physiological parameters of the patient measured with the sensor comprises an oxygen saturation of a patient when the first cuff is inflated and the sensor is positioned against the wall of the trachea.

## Patentansprüche

1. Endotracheal- oder Tracheostomietubus, umfassend:
ein rohrförmiges Element, das sich von einem proximalen Ende zu einem distalen Ende erstreckt und eine Außenfläche und eine Innenfläche des rohrförmigen Elements definiert;
eine erste Manschette, die um einen Abschnitt der Außenfläche des rohrförmigen Elements positioniert ist, wobei die erste Manschette eine Innenfläche und eine Außenfläche der ersten Manschette definiert;
einen Sensor, der an oder benachbart zu der Außenfläche der ersten Manschette angeordnet ist; und
eine zweite Manschette, die über dem Sensor positioniert ist, die nur einen Abschnitt bedeckt und nicht den gesamten Flächenbereich der ersten Manschette umschließt oder bedeckt, und
wobei der Sensor zwischen der ersten und der zweiten Manschette angeordnet ist.

2. Endotrachealtubus nach Anspruch 1, wobei die Innenfläche der ersten Manschette zu der Außenfläche des rohrförmigen Elements weist und die Außenfläche der ersten Manschette weg von dem rohrförmigen Element weist und/oder der Endotrachealtubus einen ersten Aufblasschlauch umfasst, der sich durch das rohrförmige Element und in Kommunikation mit einem Inneren der ersten Manschette zum selektiven Aufblasen und Entleeren der ersten Manschette erstreckt.

3. Endotrachealtubus nach Anspruch 1, wobei:
die erste Manschette an die Außenfläche des rohrförmigen Elements an oder nahe dem distalen Ende des rohrförmigen Elements gekoppelt ist; oder
die erste Manschette aufblasbar ist; oder
die zweite Manschette nicht aufblasbar ist.

4. Endotrachealtubus nach Anspruch 2, wobei:
die zweite Manschette aufblasbar ist und der erste Aufblasschlauch in Kommunikation mit einem Inneren der zweiten Manschette zum selektiven Aufblasen und Entleeren der zweiten Manschette ist; oder
die zweite Manschette aufblasbar ist und der Endotrachealtubus einen zweiten Aufblasschlauch umfasst, der sich durch das rohrförmige Element und in Kommunikation mit einem Inneren der zweiten Manschette zum selektiven Aufblasen und Entleeren der zweiten Manschette unabhängig von der ersten Manschette erstreckt.

5. Endotrachealtubus nach Anspruch 1, wobei die zweite Manschette eine Innenfläche und eine Außenfläche der zweiten Manschette definiert, und wobei der Sensor zwischen der ersten und der zweiten Manschette angeordnet ist, sodass die Innenfläche der zweiten Manschette unmittelbar benachbart zu dem Sensor positioniert ist, und wobei die zweite Manschette dazu konfiguriert ist, sich über den Sensor während des Aufblasens der ersten Manschette zu erstrecken, und wobei die zweite Manschette ferner dazu konfiguriert ist, eine Luftschicht zwischen dem Sensor und der zweiten Manschette zu verhindern oder zu minimieren.

6. Endotrachealtubus nach Anspruch 1, wobei:
der Sensor physisch an der Außenfläche der ersten Manschette ohne physische Anbringung an der zweiten Manschette angebracht ist; oder
der Sensor physisch an der Innenfläche der zweiten Manschette ohne physische Anbringung an der ersten Manschette angebracht ist; oder
der Sensor physisch an der Außenfläche der ersten Manschette und der Innenfläche der zweiten Manschette angebracht ist; oder
die zweite Manschette über einem gesamten Flächenbereich der Außenfläche der ersten Manschette angeordnet ist.

7. Endotrachealtubus nach Anspruch 1, wobei die zweite Manschette einen Umfangsrand umfasst, der an die Außenfläche der ersten Manschette gekoppelt ist, um eine Tasche zu bilden, und wobei der Abschnitt des Flächenbereichs weniger als 50 % ist, und wobei der Abschnitt des Flächenbereichs weniger als 25 % ist.

8. Endotrachealtubus nach Anspruch 1, wobei:
der Sensor ein einzelnes Pulsoximeter umfasst; oder
der Sensor mehrere verschiedene Arten von Sensoren umfasst.

9. Endotrachealtubus nach Anspruch 1, wobei der Sensor dazu konfiguriert ist, sich radial nach außen weg von dem rohrförmigen Element während des Aufblasens der ersten Manschette zu bewegen, und wobei optional das distale Ende des rohrförmigen Elements dazu konfiguriert ist, innerhalb einer Trachea eines Patienten positioniert zu sein, und Aufblasen der ersten Manschette den Sensor radial nach außen weg von dem rohrförmigen Element und gegen eine Wand der Trachea bewegt.

10. Endotrachealtubus nach Anspruch 1, wobei die erste Manschette und die zweite Manschette aus einem gleichen Material hergestellt sind.

11. Endotrachealtubus nach Anspruch 1, wobei die erste Manschette und die zweite Manschette aus einem biokompatiblen Material hergestellt sind.

12. Endotrachealtubus nach Anspruch 1, umfassend einen elektrischen Leiter, der an den Sensor gekoppelt ist und sich durch einen Abschnitt des rohrförmigen Elements erstreckt.

13. Endotrachealtubusanordnung zum Messen von einem oder mehreren Vitalzeichen oder physiologischen Parametern eines Patienten, wobei die Anordnung Folgendes umfasst:
ein rohrförmiges Element, das sich von einem proximalen Ende zu einem distalen Ende erstreckt und eine Außenfläche definiert, (i) eine erste Manschette, die um einen Abschnitt der Außenfläche des rohrförmigen Elements positioniert ist, wobei die erste Manschette eine Innenfläche und eine Außenfläche der ersten Manschette definiert, (ii) einen Sensor, der an oder benachbart zu der Außenfläche der ersten Manschette angeordnet ist, und (iii) eine zweite Manschette, die über dem Sensor positioniert ist, wobei die zweite Manschette über nur einem Abschnitt eines Flächenbereichs der Außenfläche angeordnet ist und nicht den gesamten Flächenbereich der ersten Manschette umschließt oder bedeckt, wobei der Sensor zwischen der ersten und der zweiten Manschette angeordnet ist;
wobei die erste Manschette dazu konfiguriert ist, zu expandieren, um den Sensor radial nach außen weg von dem rohrförmigen Element und gegen eine Wand der Trachea zu bewegen, wenn der Endotrachealtubus in die Trachea eingeführt ist; und
wobei der Sensor dazu konfiguriert ist, ein oder mehrere Vitalzeichen oder physiologische Parameter des Patienten zu messen.

14. Endotrachealtubusanordnung nach Anspruch 13, wobei:
die erste Manschette dazu konfiguriert ist, sich mit einem Fluid aufzublasen, um den Sensor radial nach außen weg von dem rohrförmigen Element und gegen die Wand der Trachea zu bewegen, wobei die erste Manschette eine Dichtung mit der Wand der Trachea bildet; oder
die zweite Manschette dazu konfiguriert ist, sich mit einem Fluid aufzublasen, um den Sensor radial nach außen weg von dem rohrförmigen Element und gegen die Wand der Trachea zu bewegen.

15. Endotrachealtubusanordnung nach Anspruch 13, wobei:
die zweite Manschette eine Innenfläche und eine Außenfläche der zweiten Manschette definiert, und wobei der Sensor zwischen der ersten Manschette und der zweiten Manschette angeordnet ist, sodass die Innenfläche der zweiten Manschette unmittelbar benachbart zu dem Sensor positioniert ist; oder
das/der eine oder die mehreren Vitalzeichen oder physiologischen Parameter des Patienten, die mit dem Sensor gemessen werden, eine Sauerstoffsättigung eines Patienten umfassen, wenn die erste Manschette aufgeblasen ist und der Sensor gegen die Wand der Trachea positioniert ist.

## Revendications

1. Tube endotrachéal ou de trachéotomie, comprenant :
un élément tubulaire s'étendant à partir d'une extrémité proximale jusqu'à une extrémité distale, et définissant une surface externe et une surface interne de l'élément tubulaire ;
un premier manchon positionné autour d'une partie de la surface externe de l'élément tubulaire, le premier manchon définissant une surface interne et une surface externe du premier manchon ;
un capteur disposé sur la surface externe du premier manchon ou à proximité de celle-ci ; et
un second manchon, positionné sur le capteur, qui ne recouvre qu'une partie et n'entoure ni ne recouvre toute la surface du premier manchon et,
ledit capteur étant disposé entre les premier et second manchons.

2. Tube endotrachéal selon la revendication 1, ladite surface interne du premier manchon étant tournée vers la surface externe de l'élément tubulaire, et ladite surface externe du premier manchon étant tournée à l'opposé de l'élément tubulaire, et/ou ledit tube endotrachéal comprenant un premier tube de gonflage s'étendant à travers l'élément tubulaire et en communication avec l'intérieur du premier manchon pour gonfler et dégonfler de manière sélective le premier manchon.

3. Tube endotrachéal selon la revendication 1 :
ledit premier manchon étant couplé à la surface externe de l'élément tubulaire au niveau de l'extrémité distale de l'élément tubulaire ou près de celle-ci ; ou
ledit premier manchon étant gonflable ; ou
ledit second manchon étant non gonflable.

4. Tube endotrachéal selon la revendication 2 :
ledit second manchon étant gonflable, et ledit premier tube de gonflage étant en communication avec l'intérieur du second manchon pour gonfler et dégonfler de manière sélective le second manchon ; ou
ledit second manchon étant gonflable, et ledit tube endotrachéal comprenant un second tube de gonflage s'étendant à travers l'élément tubulaire et en communication avec l'intérieur du second manchon pour gonfler et dégonfler de manière sélective le second manchon indépendamment du premier manchon.

5. Tube endotrachéal selon la revendication 1, ledit second manchon définissant une surface interne et une surface externe du second manchon, et ledit capteur étant disposé entre les premier et second manchons de sorte que la surface interne du second manchon soit positionnée immédiatement à proximité du capteur, et ledit second manchon étant conçu pour s'étirer sur le capteur pendant le gonflage du premier manchon, et ledit second manchon étant en outre conçu pour empêcher ou minimiser une couche d'air entre le capteur et le second manchon.

6. Tube endotrachéal selon la revendication 1 :
ledit capteur étant physiquement fixé à la surface externe du premier manchon sans fixation physique au second manchon ; ou
ledit capteur étant physiquement fixé à la surface interne du second manchon sans fixation physique au premier manchon ; ou
ledit capteur étant physiquement fixé à la surface externe du premier manchon et à la surface interne du second manchon ; ou
ledit second manchon étant disposé sur toute la surface de la surface externe du premier manchon.

7. Tube endotrachéal selon la revendication 1, ledit second manchon comprenant un bord de périmètre qui est couplé à la surface externe du premier manchon de manière à former une poche, et ladite partie de la zone de surface représentant moins de 50 %, et ladite partie de la zone de surface représentant moins de 25 %.

8. Tube endotrachéal selon la revendication 1 :
ledit capteur comprenant un seul sphygmo-oxymètre ; ou
ledit capteur comprenant plusieurs types différents de capteurs.

9. Tube endotrachéal selon la revendication 1, ledit capteur étant conçu pour se déplacer radialement vers l'extérieur en s'éloignant de l'élément tubulaire pendant le gonflage du premier manchon, et éventuellement, ladite extrémité distale de l'élément tubulaire étant conçue pour être positionnée à l'intérieur de la trachée d'un patient, et ledit gonflage du premier manchon déplaçant le capteur radialement vers l'extérieur en s'éloignant de l'élément tubulaire et contre une paroi de la trachée.

10. Tube endotrachéal selon la revendication 1, ledit premier manchon et ledit second manchon étant fabriquées à partir d'un même matériau.

11. Tube endotrachéal selon la revendication 1, ledit premier manchon et ledit second manchon étant fabriqués à partir d'un matériau biocompatible.

12. Tube endotrachéal selon la revendication 1, comprenant un conducteur électrique couplé au capteur et s'étendant à travers une partie de l'élément tubulaire.

13. Ensemble tube endotrachéal destiné à mesurer un ou plusieurs signes vitaux ou paramètres physiologiques d'un patient, l'ensemble comprenant :
un élément tubulaire s'étendant à partir d'une extrémité proximale jusqu'à une extrémité distale, et définissant une surface externe, (i) un premier manchon positionné autour d'une partie de la surface externe de l'élément tubulaire, le premier manchon définissant une surface interne et une surface externe du premier manchon, (ii) un capteur disposé sur la surface externe du premier manchon ou à proximité de celle-ci, et (iii) un second manchon positionné sur le capteur, ledit second manchon étant disposé sur seulement une partie d'une zone de surface de la surface externe et n'entourant pas ni ne recouvrant toute la zone de surface du premier manchon, ledit capteur étant disposé entre les premier et second manchons ;
ledit premier manchon étant conçu pour se dilater de manière à déplacer le capteur radialement vers l'extérieur en s'éloignant de l'élément tubulaire et contre une paroi de la trachée lorsque le tube endotrachéal est inséré dans la trachée ; et
ledit capteur étant conçu pour mesurer un ou plusieurs signes vitaux ou paramètres physiologiques du patient.

14. Ensemble tube endotrachéal selon la revendication 13 :
ledit premier manchon étant conçu pour se gonfler avec un fluide de manière à déplacer le capteur radialement vers l'extérieur en s'éloignant de l'élément tubulaire et contre la paroi de la trachée, ledit premier manchon formant un joint d'étanchéité avec la paroi de la trachée ; ou
ledit second manchon étant conçu pour se gonfler avec un fluide de manière à déplacer le capteur radialement vers l'extérieur en s'éloignant de l'élément tubulaire et contre la paroi de la trachée.

15. Ensemble tube endotrachéal selon la revendication 13 :
ledit second manchon définissant une surface interne et une surface externe du second manchon, et ledit capteur étant disposé entre le premier manchon et le second manchon de sorte que la surface interne du second manchon soit positionnée immédiatement à proximité du capteur ;
ou
lesdits un ou plusieurs signes vitaux ou paramètres physiologiques du patient mesurés avec le capteur comprenant la saturation en oxygène d'un patient lorsque le premier manchon est gonflé et que le capteur est positionné contre la paroi de la trachée.
